# EUROPEAN PATENT APPLICATION

(11) **EP 4 834 278 A1**
(43) Date of publication of application: **04.11.2026**
(21) Application number: 24910860.6
(22) Date of filing: 18.12.2024
(51) Int. Cl.: A61F 2/07

(54) **COVERED STENT AND STENT DELIVERY SYSTEM**

(30) Priority: 27.12.2023 CN 202311838049
(71) Applicant: Lifetech Scientific (Shenzhen) Co., Ltd., Shenzhen, Guangdong 518063 (CN)
(72) Inventor: TANG, Jiangfeng, Shenzhen, Guangdong 518063 (CN); TANG, Benping, Shenzhen, Guangdong 518063 (CN)
(74) Representative: Prinz & Partner mbB
(86) International application number: PCT/CN2024/140153
(87) International publication number: WO 2025/139929

(57) **Abstract**

Provided are a covered stent (100) and a stent delivery system (200). The covered stent (100) includes a main stent (10) provided with a tubular body. Along an axial direction, the main stent (10) includes a proximal support section (1) and an aneurysm sac covered section (3). The aneurysm sac covered section (3) is internally provided with a first embedded stent (4) and a second embedded stent (5) arranged radially. A distal end of the aneurysm sac covered section (3) is provided with an opening (32) in communication with the outside. Distal openings of the first embedded stent (4) and the second embedded stent (5) are both in communication with the opening (32). The first embedded stent (4) has higher support strength than the second embedded stent (5). By arranging the first embedded stent (4) with relatively high support strength, patency of an external iliac channel corresponding to the first embedded stent (4) is ensured at least when the covered stent is compressed. Further, extended covering membranes of the first embedded stent (4) and the second embedded stent (5) can ensure sealing integrity at an attachment gap (4021) after being sutured to the main stent (10), thereby preventing endoleaks at the embedded stents.

## Description

### Technical Field

The present invention relates to the technical field of medical devices, and in particular, to a covered stent and a stent delivery system.

### Background Art

Iliac artery, as one of the vital vascular systems in the human body, originates from the terminal portion of the abdominal aorta. It bifurcates into the left and right common iliac arteries, passes at the level of the fourth lumbar vertebra, extends down along the spine on the medial side of the psoas major muscle, and ultimately bifurcates into the internal iliac artery and the external iliac artery. The internal iliac artery is responsible for supplying oxygen and nutrients to the pelvis and lower limbs, whereas the external iliac artery supplies blood to the ilium and its surrounding tissues. However, existing iliac artery bifurcation stents suffer from a series of drawbacks, such as the susceptibility of the ostium of the internal iliac artery to compression-induced occlusion, the tendency of the internal iliac branch to tortuosity and kinking that result in lumen obstruction, and risk of ostial blockage of the internal iliac branch caused by slight displacement or rotation of the stent during implantation.

To solve the above problems, in the prior art, an embedded branch stent is specifically designed, in which a first branch is configured to connect the external iliac artery, and the second branch is configured to connect the internal iliac artery. This innovative structural design has broad patient applicability, can extend to the proximal side of the iliac bifurcation without being limited by the length of the common iliac artery, and is suitable for treating internal and external iliac arteries of various diameters. This innovative structure allows the stent to be more easily aligned during implantation, avoids the difficulties commonly encountered with the iliac bifurcation stent, and enables easier implantation of the internal and external iliac stents.

However, challenges remain in the design of the embedded branch stent, especially the potential risk of endoleaks at the site of the embedded stent.

### Summary of the Invention

Based on this, it is necessary to provide a new covered stent and a stent delivery system, to at least solve the problem of endoleaks associated with an embedded stent within a main stent.

A covered stent includes a main stent provided with a tubular body. The main stent includes a proximal support section and an aneurysm sac covered section, and a distal end of the proximal support section is connected to a proximal end of the aneurysm sac covered section; the proximal support section includes a support corrugated ring, the aneurysm sac covered section is internally provided with a first embedded stent and a second embedded stent arranged radially, a distal end of the aneurysm sac covered section is provided with an opening in communication with the outside, and distal openings of the first embedded stent and the second embedded stent are both in communication with the opening; and the first embedded stent has higher support strength than the second embedded stent.

In one embodiment, each of the first embedded stent and the second embedded stent includes a mesh main body, and the first embedded stent has a larger wire diameter than the second embedded stent and/or a higher grid density than the second embedded stent on the mesh main body.

In one embodiment, distal ends of the first embedded stent and the second embedded stent are respectively provided with a first distal oblique opening and a second distal oblique opening, and the first distal oblique opening and the second distal oblique opening face away from each other.

In one embodiment, proximal ends of the first embedded stent and the second embedded stent are respectively provided with a first proximal flat opening and a second proximal flat opening; or proximal ends of the first embedded stent and the second embedded stent are respectively provided with a first proximal oblique opening and a second proximal oblique opening, and the first proximal oblique opening and the second proximal oblique opening face each other.

In one embodiment, the covered stent further includes a transition section connected between the proximal support section and the aneurysm sac covered section, and a transition stent is arranged on the transition section.

In one embodiment, a first covering membrane is arranged on a surface of the first embedded stent, a second covering membrane is arranged on a surface of the second embedded stent, the first covering membrane and the second covering membrane at least partially extend outward from proximal openings of the first embedded stent and the second embedded stent, respectively, to form connection portions, and the connection portions are connected to the aneurysm sac covered section.

In one embodiment, the connection portion of the first covering membrane is connected to the connection portion of the second covering membrane.

In one embodiment, the first covering membrane and the second covering membrane are integrally formed.

In one embodiment, the first distal oblique opening and the second distal oblique opening each include a long-axis side wall and a short-axis side wall in a circumferential direction, an axial extension length of the long-axis side wall is greater than an axial extension length of the short-axis side wall, long-axis side walls of the first embedded stent and the second embedded stent are closely attached to each other, and a hooking portion is arranged at a distal end of at least one long-axis side wall.

In one embodiment, the support corrugated ring includes at least one anchoring corrugated ring, and a plurality of anchoring barbs are circumferentially arranged on an outer side of the anchoring corrugated ring.

A stent delivery system includes the covered stent.

The present invention has the following benefits. The present invention provides the covered stent and the stent delivery system, the covered stent includes the main stent provided with the tubular body, the main stent includes the proximal support section and the aneurysm sac covered section along an axial direction, the aneurysm sac covered section is internally provided with the first embedded stent and the second embedded stent arranged radially, the distal end of the aneurysm sac covered section is provided with the opening in communication with the outside, and the distal openings of the first embedded stent and the second embedded stent are both in communication with the opening; the first embedded stent has higher support strength than the second embedded stent; by arranging the first embedded stent with relatively high support strength, patency of an external iliac channel corresponding to the first embedded stent is ensured at least when the covered stent is compressed; and further, the extended covering membranes of the first embedded stent and the second embedded stent can ensure sealing integrity at an attachment gap after being sutured to the main stent, thereby preventing endoleaks at the embedded stents.

### Brief Description of the Drawings

FIG. 1 is a schematic structural diagram of a covered stent according to Embodiment 1 of the present invention;
FIG. 2 is a schematic structural diagram of an interior of a covered stent according to Embodiment 1 of the present invention;
FIG. 3 is a schematic diagram of wire diameter distribution of a first embedded stent and a second embedded stent according to Embodiment 1 of the present invention;
FIG. 4 is a schematic diagram of grid density distribution of a first embedded stent and a second embedded stent according to Embodiment 1 and Embodiment 2 of the present invention;
FIG. 5 is a schematic diagram of proximal flat openings of a first embedded stent and a second embedded stent according to Embodiment 2 of the present invention;
FIG. 6 is a schematic diagram of developing structures of a first embedded stent and a second embedded stent according to Embodiment 2 of the present invention;
FIG. 7 is a schematic structural diagram of a hooking portion in a covered stent according to Embodiment 2 of the present invention;
FIG. 8 is a schematic diagram of a blocking member arranged on a hooking portion according to Embodiment 2 of the present invention;
FIG. 9 is a schematic diagram of a blocking member as a developing structure on a hooking portion according to Embodiment 2 of the present invention;
FIG. 10 is a schematic diagram of a transition stent as a special-shaped corrugated ring according to Embodiment 2 of the present invention;
FIG. 11 is a schematic diagram of a distal high wave of a special-shaped corrugated ring according to Embodiment 2 of the present invention;
FIG. 12 is a schematic diagram of gradual reduction of a distal high wave of a special-shaped corrugated ring according to Embodiment 2 of the present invention;
FIG. 13 is a schematic diagram of a transition stent including a corrugated ring stent according to Embodiment 2 of the present invention;
FIG. 14 is a schematic diagram of a transition stent including a mesh stent according to Embodiment 2 of the present invention;
FIG. 15 is a schematic structural diagram of a first covering membrane and a second covering membrane according to Embodiment 3 of the present invention;
FIG. 16 is a schematic structural diagram of connection portions of a first covering membrane and a second covering membrane according to Embodiment 3 of the present invention;
FIG. 17 is a top view of a covered stent when connection portions of the first covering membrane and a second covering membrane are connected to an aneurysm sac covered section according to Embodiment 3 of the present invention;
FIG. 18 is a schematic structural diagram of connecting a first covering membrane and a second covering membrane through connection portions according to Embodiment 3 of the present invention;
FIG. 19 is a top view of a covered stent when a first covering membrane and a second covering membrane are connected through connection portions and then connected to an aneurysm sac covered section according to Embodiment 3 of the present invention;
FIG. 20 is a schematic structural diagram of integrally forming a first covering membrane and a second covering membrane according to Embodiment 3 of the present invention;
FIG. 21 is a top view of a covered stent when a first covering membrane and a second covering membrane which are integrally formed are connected to an aneurysm sac covered section according to Embodiment 3 of the present invention;
FIG. 22 is a schematic structural diagram of an intermediate anchoring corrugated ring according to Embodiment 4 of the present invention; and
FIG. 23 is a schematic structural diagram of hooking a covered stent in a delivery device according to Embodiment 5 of the present invention.

### Detailed Description of the Invention

To better understand the concept of the present invention, implementations of the present invention are described in detail below with reference to the drawings, and the following specific embodiments are only part of the embodiments of the present invention, but not limitations to the present invention.

For ease of description, spatial relative relationship terms may be used herein to describe a relationship between one element or feature and another element or feature as shown in the figures. These relative relationship terms include, for example, "interior", "exterior", "inner side", "outer side", "below", "under", "above", "over", and the like. Such spatial relative relationship terms are meant to include different orientations of a device in use or operation other than orientations depicted in the figures. For example, if the device in the figures is overturned, an element described as "under" or "below" another element or feature will then be oriented as "above" or "over" another element or feature. Accordingly, an example term "below" may include both an upper orientation and a lower orientation. The device may otherwise be oriented (rotated 90 degrees or in other directions) and a spatial relative relationship descriptor used herein is interpreted accordingly.

Although terms such as first, second, and third may be used herein to describe a plurality of elements, components, areas, layers, and/or sections, these elements, components, areas, layers, and/or sections should not be limited by these terms. These terms may only be configured to distinguish one element, component, area, layer, or section from another element, component, area, layer, or section. The terms such as "first", "second", and other numerical terms, when used herein, do not imply an order or a sequence unless otherwise specified in the context. Therefore, a first element, component, area, layer, or section discussed below may be referred to as a second element, component, area, layer, or section without departing from the teachings of example implementations.

To describe the structure of the present invention more clearly, terms "proximal end" and "distal end" are defined as common terms used in the field of interventional medicine herein. Specifically, the "distal end" indicates one end of a blood vessel away from the heart, and the "proximal end" indicates one end of a blood vessel close to the heart; "axial direction" indicates a length direction, and "radial direction" indicates a direction perpendicular to the "axial direction"; and "upper end" and "lower end" are two ends relatively away from each other, and when one end is defined as the "upper end", the other end away from this end is the "lower end".

### Embodiment 1

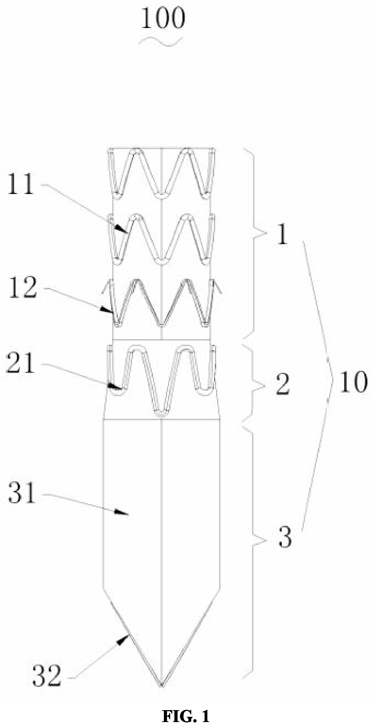

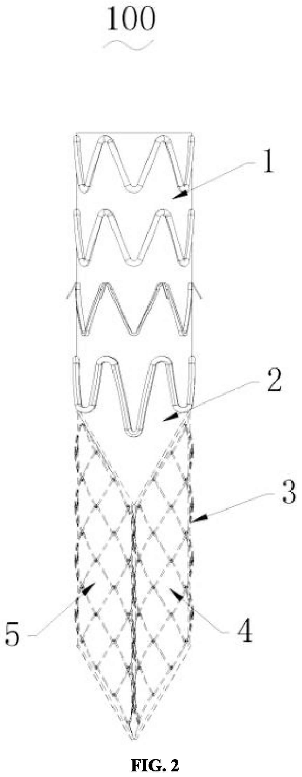

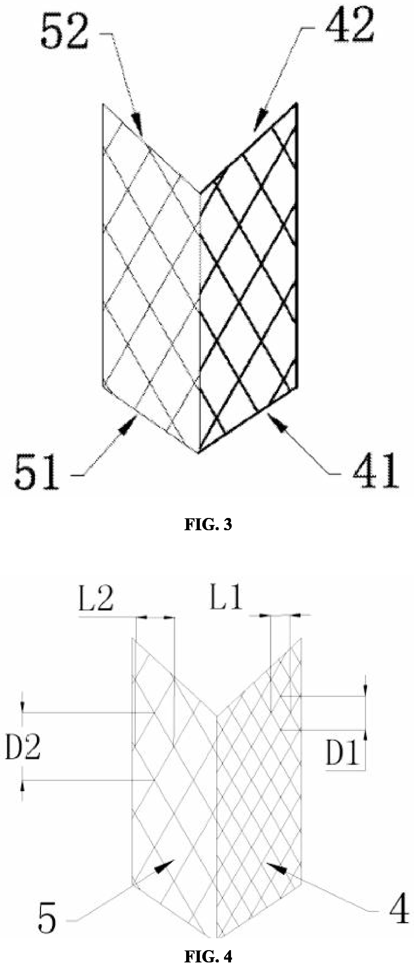

Referring to FIG. 1 and FIG. 2, the present invention provides a covered stent 100. The covered stent 100 usually includes a metallic framework and a covering membrane material. As shown in FIG. 3, the metallic framework can adopt a Z-shaped wave or a braided mesh design, and the covering membrane material has a certain blood flow isolation ability, and is combined with the metallic framework in a pressurized heating or suturing manner to form a complete covered stent 100. A proximal end of the covered stent 100 is typically placed in a common iliac artery or connected to an abdominal aortic stent, and a lumen diameter is generally matched with a diameter of the common iliac artery. In this embodiment, the covered stent 100 includes a main stent 10 provided with a tubular body, a surface covering membrane 31 is arranged on a surface of the main stent 10, the main stent 10 includes a proximal support section 1 and an aneurysm sac covered section 3 along an axial direction, a distal end of the proximal support section 1 is connected to a proximal end of the aneurysm sac covered section 3, and the surface covering membrane 31 of the proximal support section 1 and the surface covering membrane 31 of the aneurysm sac covered section 3 may be integrally formed as a single covering membrane, or may be formed by splicing a plurality of covering membranes in a suturing or bonding manner; the proximal support section includes a support corrugated ring 11, while the surface covering membrane 31 is arranged only on the aneurysm sac covered section 3, and the aneurysm sac covered section 3 is internally provided with a first embedded stent 4 and a second embedded stent 5 arranged radially, such that support corrugated rings 11 of the main stent 10 are omitted at the aneurysm sac covered section 3 to improve the flexibility of the covered stent 100 at the aneurysm sac section; and the aneurysm sac covered section 3 provides support through the first embedded stent 4 and the second embedded stent 5. In addition, when blood flows through the aneurysm sac covered section 3, the blood is divided to flow into an external iliac channel and an internal iliac channel, and the first embedded stent 4 and the second embedded stent 5 are configured to respectively allow an external iliac stent and an internal iliac stent to be implanted, to guide the blood to an external iliac artery and an internal iliac artery, isolate the blood, and prevent the blood from continuing to flow toward an aneurysm.

In some embodiments, continuing to refer to FIG. 2, the first embedded stent 4 and the second embedded stent 5 each adopt a mesh main body structure, to achieve better form supporting performance; the mesh main body structure can have better covering membrane tension, and even under a condition of a small wire diameter of a braiding wire, the mesh main body structure can still provide better covering membrane tension to maintain a form of a blood pathway; and in addition, the mesh main body can ensure that when the external iliac stent and the internal iliac stent are respectively implanted into the first embedded stent 4 and the second embedded stent 5, more contact points are present between the embedded stents and the implanted stents to provide greater frictional force, thereby effectively enhancing anchoring force and attachment force between the stents to achieve anti-migration performance of the stents.

A distal end of the aneurysm sac covered section 3 is provided with an opening 32 in communication with the outside, and distal openings of the first embedded stent 4 and the second embedded stent 5 are both in communication with the opening 32; in order to ensure that the covered stent 100 provided in the present invention preferentially maintains a form of a pathway in communication with an external iliac vessel when subjected to a pressure, in this embodiment, the first embedded stent 4 is allowed to have higher support strength than the second embedded stent 5. Herein, the support strength is manifested as the fact that an overall deformation amount of the first embedded stent 4 is smaller than an overall deformation amount of the second embedded stent 5 when the first embedded stent 4 and the second embedded stent 5 are subjected to the same pressure, to maintain a better pathway form.

In this embodiment, referring to FIG. 3 and FIG. 4, in order to allow the external iliac channel and the internal iliac channel separated by the first embedded stent 4 and the second embedded stent 5 to have a support strength distribution effect as described above, the mesh main body of the first embedded stent 4 can have a larger wire diameter than the mesh main body of the second embedded stent 5, and/or the first embedded stent 4 can have a higher grid density than the second embedded stent 5.

In one embodiment, referring to FIG. 3, the first embedded stent 4 and the second embedded stent 5 are allowed to have a uniform grid density, while the mesh main body of the first embedded stent 4 is allowed to have a larger wire diameter than the mesh main body of the second embedded stent 5, such that the first embedded stent 4 has higher support strength than the second embedded stent 5. Herein, a stent with a larger wire diameter has a lower deformation capacity, and can provide greater supporting force when stressed. Therefore, by allowing the first embedded stent 4 to have a larger wire diameter than the second embedded stent 5, when the first embedded stent 4 and the second embedded stent 5 are stressed at the same time, due to its larger wire diameter, the first embedded stent 4 has higher resistance to deformation and generates a small deformation amount, while due to its smaller wire diameter, the second embedded stent 5 has lower resistance to deformation and generates a large deformation amount, whereby a form of the first embedded stent 4 can be better maintained, to ensure that the covered stent 100 better maintains its form in the external iliac channel and further ensure blood flow to the external iliac artery after the blood passes through the covered stent 100.

In another embodiment, referring to FIG. 4, the first embedded stent 4 and the second embedded stent 5 are allowed to have a uniform wire diameter, while the mesh main body of the first embedded stent 4 is allowed to have a higher grid density than the mesh main body of the second embedded stent 5, such that the first embedded stent 4 has higher support strength than the second embedded stent 5. Herein, the first embedded stent 4 and the second embedded stent 5 each include the mesh main body, and the mesh main body has a plurality of grid structures, wherein a high grid density means increase of a number of required supporting wires, a single grid with a smaller area can withstand a higher pressure, and the higher grid density can provide higher support strength. In this embodiment, the first embedded stent 4 and the second embedded stent 5 each have a diamond grid structure, the first embedded stent 4 has a first diamond grid, the second embedded stent 5 has a second diamond grid, and the first diamond grid and the second diamond grid each have an upper vertex and a lower vertex in an axial direction, and have a left vertex and a right vertex in a radial direction; a gap between the upper vertex and the lower vertex of the first diamond grid is D1, and a gap between the left vertex and the right vertex is L1; a gap between the upper vertex and the lower vertex of the second diamond grid is D2, and a gap between the left vertex and the right vertex is L2; and preferably, both D1 and L1 may be smaller than D2 and L2, such that an area occupied by a single first diamond grid is smaller than an area occupied by a single second diamond grid, whereby in the same stent deployment area, the first embedded stent 4 has a higher grid density and provides higher support strength than the second embedded stent 5.

In some embodiments, further referring to FIG. 4, at least L1 of the first diamond grid is allowed to be smaller than L2 of the second diamond grid, such that the density of the first diamond grid is changed only in a circumferential direction of the first embedded stent 4. In this way, the density of the first diamond grid can be increased at least in a radial direction, thereby achieving an effect of enhancing the support strength in the radial direction.

In one embodiment, the first embedded stent 4 can be allowed to have a larger wire diameter than the second embedded stent 5 and a higher grid density than the second embedded stent 5 at the same time. In this way, by setting wire diameters and grid densities of the first embedded stent 4 and the second embedded stent 5 at the same time, the first embedded stent 4 and the second embedded stent 5 can have a difference in support strength, and at the same time, relatively reasonable wire diameter size and grid density distribution can be achieved, to prevent a condition that excessively large or small wire diameter and excessively high or low grid density settings cause excessively high or low flexibility of the first embedded stent 4 and the second embedded stent 5 to affect folding and unfolding performance of the covered stent 100 and result in assembly and release difficulties of the covered stent 100 in a sheath.

In this embodiment, the support strength is specifically manifested as a deformation amount of an entire tubular lumen after the first embedded stent 4 and the second embedded stent 5 are stressed, that is, under the same stress condition, outer side walls of the first embedded stent 4 and the second embedded stent 5 are compressed by a radial force tester using the same force (this force is required to enable the first embedded stent 4 and the second embedded stent 5 to generate a certain amount of deformation), and radial cross-sectional areas of the first embedded stent 4 and the second embedded stent 5 after compression can be measured and calculated. Herein, after compression, the stent with a larger total cross-sectional area measured has higher support strength, and the stent with a smaller total cross-sectional area has lower support strength. In some embodiments, the first embedded stent 4 and the second embedded stent 5 can be compressed by a flat dynamometer, and force required when the same deformation amount is achieved through compression is measured; when the measured force is large, the support strength is high; and when the measured force is small, the support strength is low. In this embodiment, the force measured on the first embedded stent 4 is always greater than the force measured on the second embedded stent 5.

### Embodiment 2

In this embodiment, continuing to refer to FIG. 4, structures of a main stent 10, a first embedded stent 4, and a second embedded stent 5 of the covered stent 100 are substantially the same as those in Embodiment 1, and a difference lies in that an opening 32 located at a distal end of the covered stent 100 is configured as an oblique opening. Specifically, distal ends of the first embedded stent 4 and the second embedded stent 5 are respectively provided with a first distal oblique opening 41 and a second distal oblique opening 51, and the first distal oblique opening 41 and the second distal oblique opening 51 face away from each other. Herein, distal openings of the first embedded stent 4 and the second embedded stent 5 are openings for introducing an external iliac stent and an internal iliac stent, and the distal openings of the two embedded stents are both configured as oblique openings due to the following reasons: first, a size of the opening 32 is increased when the external iliac stent and the internal iliac stent are introduced, thereby reducing a difficulty in introducing the stents; and second, since connection to an external iliac artery and an internal iliac artery is established by additionally introducing the external iliac stent and the internal iliac stent in the present invention, the first distal oblique opening 41 and the second distal oblique opening 51 face away from each other to allow the two introduced stents to be less bound by the covered stent 100 in opposite directions, whereby the external iliac stent and the internal iliac stent are easier to separate from each other after connection, and interaction between the two stents is avoided.

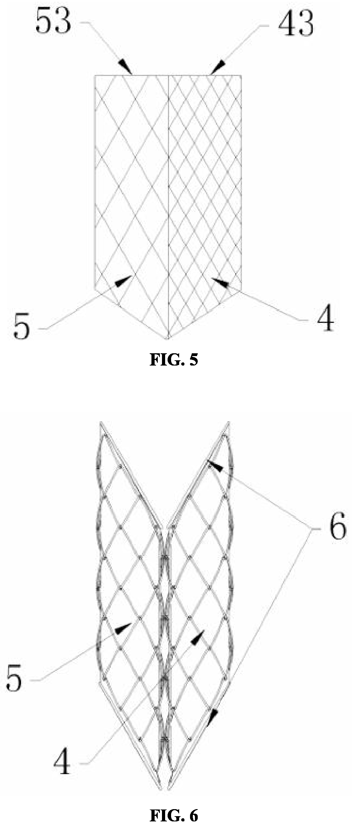

Referring to FIG. 4 and FIG. 5, proximal openings of the first embedded stent 4 and the second embedded stent 5 may be configured as flat openings or oblique openings. Specifically, proximal ends of the first embedded stent 4 and the second embedded stent 5 may be respectively provided with a first proximal flat opening 43 and a second proximal flat opening 53. Herein, in order to form a double-layer stent when the first embedded stent 4 and the second embedded stent 5 are arranged and simultaneously reduce an overall volume of the stent after compression and sheathing, the first proximal flat opening 43 and the second proximal flat opening 53 can be arranged in a staggered manner in an axial direction, to be located at different axial positions after compression, thereby avoiding an overlarge volume and a sheathing trouble due to stacking at one axial position.

In another embodiment, further referring to FIG. 4, the proximal ends of the first embedded stent 4 and the second embedded stent 5 may be respectively provided with a first proximal oblique opening 42 and a second proximal oblique opening 52, and the first proximal oblique opening 42 and the second proximal oblique opening 52 face each other. Herein, the two oblique openings form a V-shaped cross section on an axial tangent plane, and the first proximal oblique opening 42 and the second proximal oblique opening 52 are blood inflow openings in an aneurysm sac covered section 3, and are configured as oblique opening structures to increase areas of the blood inflow openings, thereby ensuring patency of blood flow. Further, the first proximal oblique opening 42 and the second proximal oblique opening 52 face each other, such that a side wall with a higher oblique opening is closely attached to a side wall of the aneurysm sac covered section 3, to prevent vibration or swing during blood flushing and avoid reduction of the patency of the blood flow. In some embodiments, the first proximal oblique opening 42 and the first distal oblique opening 41, as well as the second proximal oblique opening 52 and the second distal oblique opening 51 may have the same inclination angle, to form a parallel opening structure, to increase versatility of the first embedded stent 4 and the second embedded stent 5. In other embodiments, the arrangement of the first proximal oblique opening 42 and the second proximal oblique opening 52 enables long-axis side walls and short-axis side walls of the first embedded stent 4 and the second embedded stent 5 to be staggered. Consequently, when the covered stent 100 in the present invention is compressed and placed into a delivery sheath, the staggered configuration of the long-axis side walls and the short-axis side walls enables the first embedded stent 4 and the second embedded stent 5 to have smaller folded volumes and to be more easily delivered into the delivery sheath.

Herein, the arrangement of the first proximal oblique opening 42 and the second proximal oblique opening 52 may also provide a larger access opening for other stents during implantation, thereby reducing a difficulty of implantation and improving surgical efficiency.

Referring to FIG. 6, in order to easily identify positions of the first embedded stent 4 and the second embedded stent 5 in a blood vessel after the covered stent 100 is implanted into a human body, developing structures 6 are respectively arranged at the proximal openings and the distal openings of the first embedded stent 4 and the second embedded stent 5. Herein, the developing structures 6 may be single developing rings which match shapes of the proximal openings and the distal openings of the first embedded stent 4 and the second embedded stent 5, and are connected to edges of the proximal openings and the distal openings in a braiding or winding manner, wherein the developing rings may be made of a platinum wire or tantalum metal.

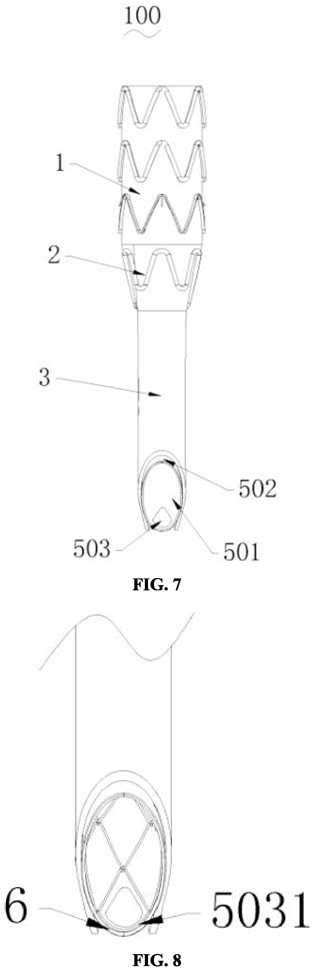

Whereas in some other embodiments, referring to FIG. 7, inclination angles of the first distal oblique opening 41 and the second distal oblique opening 51 may be larger than inclination angles of the first proximal oblique opening 42 and the second proximal oblique opening 52. Herein, since the first distal oblique opening 41 and the second distal oblique opening 51 face away from each other, a V-shaped tip protruding toward a distal end is formed at a mutual attachment position of the two stents, and the V-shaped tip may be configured to be connected to a delivery device in a hooking manner during delivery of the covered stent 100 of the present invention in the delivery device, to facilitate push and control. Specifically, the first distal oblique opening 41 and the second distal oblique opening 51 each include a long-axis side wall 501 and a short-axis side wall 502 in a circumferential direction, to form an oblique opening structure, an axial length of the long-axis side wall 501 is longer than an axial length of the short-axis side wall 502, long-axis side walls 501 of the first embedded stent 4 and the second embedded stent 5 are closely attached to each other to form a V-shaped tip at the distal end, and an axial length of the V-shaped tip is longer than axial lengths of any other positions at the distal end of the covered stent 100. Therefore, a hooking portion 503 is arranged at a distal end of the long-axis side wall 501, and the hooking portion 503 can be better hooked by a hook 2001, and the hook 2001 after hooking is prevented from affecting or being in contact with other positions of the covered stent 100. The inclination angles of the first distal oblique opening 41 and the second distal oblique opening 51 may be configured as 30° to 60°; and due to a matching requirement of a clamping and releasing structure at a distal end of the delivery device, the inclination angle of less than 30° may cause the delivery device to be unable to effectively clamp the covered stent 100, thereby affecting proceeding of surgery. On the contrary, if the inclination angle is greater than 60°, that is, an excessive angle, the stent may extend unnecessarily to a position of the external iliac artery, and implantation of the external iliac stent may be hindered in view of a small diameter of a blood vessel of the external iliac artery, thereby negatively affecting long-term patency of the stent. In addition, unnecessary extension of the stent increases a difficulty of surgery, which may impede the release of the covered stent 100 and thus affect the smooth progress of the surgery.

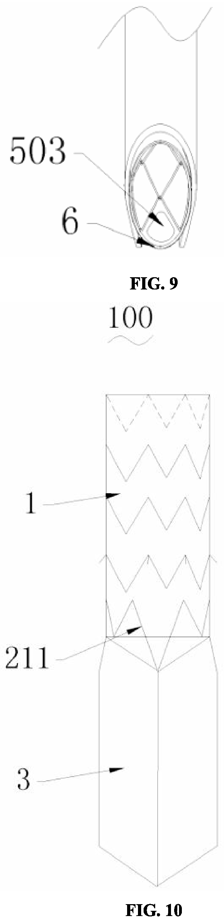

Referring to FIG. 8 and FIG. 9, herein, the hooking portion 503 may be arranged only on the long-axis side wall 501 of the first distal oblique opening 41 of the mesh main body of the first embedded stent 4, or only on the long-axis side wall 501 of the second distal oblique opening 51 of the mesh main body of the second embedded stent 5; and a hooking portion 503 is arranged at the distal oblique opening of at least one embedded stent to form a hooking position for the hook. In some embodiments, the hooking portion 503 may also be formed by grid structures at the most distal ends of the long-axis side wall 501 of the first distal oblique opening 41 and the long-axis side wall 501 of the second distal oblique opening 51 on the mesh main bodies of the first embedded stent 4 and the second embedded stent 5, and a distal end of the hooking portion 503 includes at least one blocking member 5031 for hooking, wherein the blocking member 5031 may be a wire of the grid structure, and may also be a developing ring at the first distal oblique opening 41 and the second distal oblique opening 51, and a number of stents at this position may be decreased only through a blocking effect provided by the developing ring, such that the stents are more easily released at this position. Further, in order to ensure a better hooking effect of the hooking portion 503 and the hook 2001 and ensure that the hook is not easy to detach, the grid structures at the most distal ends of the long-axis side wall 501 of the first distal oblique opening 41 and the long-axis side wall 501 of the second distal oblique opening 51 are configured as hollowed-out structures, that is, a first covering membrane 401 and a second covering membrane 504 are not arranged, such that the hook 2001 can completely pass through the grid structures of the hooking portion 503 to form hooking, to avoid accidental detachment after hooking connection.

In this embodiment, further referring to FIG. 1 and FIG. 10, because the first embedded stent 4 and the second embedded stent 5 in a proximal support section 1 and the aneurysm sac covered section 3 adopt different stent structures, structural abrupt changes and faults occur at connection positions of different sections, which is not conducive to long-term use of the stent. In order to allow a connection transition between the proximal support section 1 and the aneurysm sac covered section 3 of the covered stent 100 to be smoother, a transition section 2 is arranged between the proximal support section 1 and the aneurysm sac covered section 3 of the main stent 10, a transition stent 21 is arranged on the transition section 2, and the transition section 2 and the transition stent 21 are arranged between the proximal support section 1 and the aneurysm sac covered section 3 and configured to transition the two sections of the main stent 10. When the proximal openings of the first embedded stent 4 and the second embedded stent 5 are respectively a first proximal flat opening 43 and a second proximal flat opening 53, the transition stent 21 may be one of support corrugated rings 11 of the proximal support section 1, a proximal end of the transition stent 21 is flush with the distal flat opening of the proximal support section 1, and a distal end of the transition stent 21 is flush with the proximal flat openings of the first embedded stent 4 and the second embedded stent 5. Herein, because a surface covering membrane 31 of the aneurysm sac covered section 3 is closely attached to outer surfaces of the first embedded stent 4 and the second embedded stent 5 after being sutured onto the first embedded stent 4 and the second embedded stent 5, the first embedded stent 4 and the second embedded stent 5 have a longer length direction and a shorter width direction after being arranged side by side; and in the width direction, a width of the aneurysm sac covered section 3 is smaller than a diameter of the proximal support section 1, so the transition stent 21 has a circular cross section at the proximal end, while the distal end has a flat strip-shaped cross section with a reduced width on at least one side, and the transition stent 21 has a structure gradually reducing from the proximal end to the distal end.

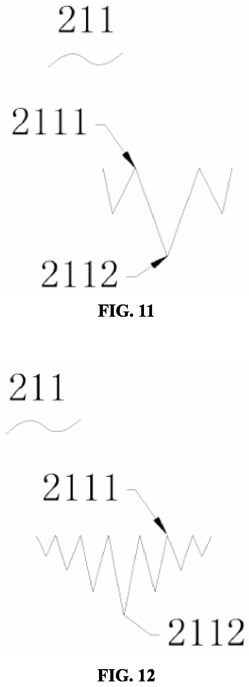

In another embodiment, continuing to refer to FIG. 10 and FIG. 11, when the proximal openings of the first embedded stent 4 and the second embedded stent 5 are respectively a first proximal oblique opening 42 and a second proximal oblique opening 52, the two oblique openings form a V-shaped cross section on an axial tangent plane, and a no-support area is formed at this position on the aneurysm sac covered section 3, wherein a proximal end of a transition stent 21 is flush with a distal flat opening of the proximal support section 1, and a distal end of the transition stent 21 is flush with proximal V-shaped double oblique openings which are jointly formed by the first embedded stent 4 and the second embedded stent 5. Herein, the transition stent 21 can support a surface covering membrane 31 of the no-support area formed between the double oblique openings, to avoid a condition that the aneurysm sac covered section 3 lacks a support structure at this position to cause collapse or poor release; and the transition stent 21 may be a special-shaped corrugated ring 211, and a distal end of the special-shaped corrugated ring 211 extends into the no-support area of the aneurysm sac covered section 3. Specifically, a proximal end of the special-shaped corrugated ring 211 has uniform proximal waves 2111 at the same height, the distal end includes a plurality of distal high waves 2112 at unequal heights, the distal high waves 2112 protrude toward the distal end, and crests are connected to the first proximal oblique opening 42 and the second proximal oblique opening 52 of the first embedded stent 4 and the second embedded stent 5, to support the no-support area, wherein at least two distal high waves 2112 of the special-shaped corrugated ring 211 are arranged, and the two distal high waves 2112 are symmetrically arranged on two sides of the distal end along a diameter of the special-shaped corrugated ring 211, and the crests of the two distal high waves 2112 are close to bottoms of the V-shaped double oblique openings.

Further, referring to FIG. 12, a plurality of distal high waves 2112 of the special-shaped corrugated ring 211 may be arranged, and a mountain-like structure having a highest wave height in the middle and gradually reduced wave heights toward two sides may be formed on each of two opposite sides of the distal end of the special-shaped corrugated ring 211, to match a shape of the no-support area. The special-shaped corrugated ring 211 can avoid a condition that the no-support area lacks a support structure to cause local collapse or poor release to affect patency of blood. In addition, a structure of the special-shaped corrugated ring 211 extending between a distal end of the proximal support section 1 and the aneurysm sac covered section 3 can allow the covered stent 100 to have sufficient connection force between the proximal support section 1 and the aneurysm sac covered section 3, and stent integrity is higher, thereby avoiding bending at a transition position between the aneurysm sac covered section 3 and the proximal support section 1.

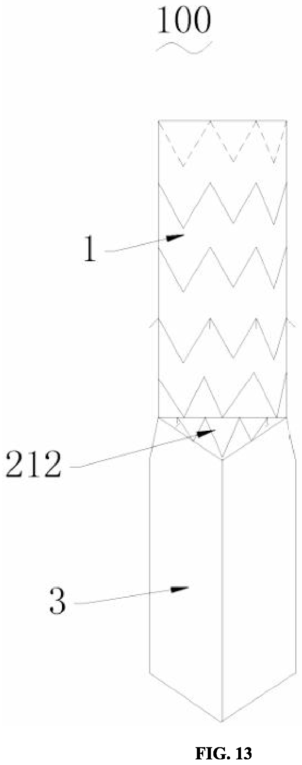

In some embodiments, referring to FIG. 13 to FIG. 14, the transition stent 21 includes a corrugated ring stent 212 and/or a mesh stent 213, wherein when proximal openings of the first embedded stent 4 and the second embedded stent 5 are respectively a first proximal flat opening 43 and a second proximal flat opening 53, the transition stent 21 may be an annular corrugated ring stent 212; when the proximal openings of the first embedded stent 4 and the second embedded stent 5 are respectively a first proximal oblique opening 42 and a second proximal oblique opening 52, the transition stent 21 may include the corrugated ring stent 212, such as the aforementioned special-shaped corrugated ring, which is not be repeatedly described herein; the transition stent may also simultaneously include the corrugated ring stent 212 and a mesh stent 213, and a form adopting the corrugated ring stent 212 is similar to the structure of the support corrugated ring 11 of the proximal support section 1, such that the transition stent has a better connection with the proximal support section 1 at this position, thereby improving the overall flexibility of the covered stent 100; and if the mesh stent 213 is adopted, the mesh stent 213 has a mesh braided structure or a mesh cutting structure, and the structure adopting the mesh stent 213 is similar to the structures of the first embedded stent 4 and the second embedded stent 5, such that the integrity of the aneurysm sac section is higher. In addition, support tension of a mesh braided stent is greater, such that an inner wall is smoother to further ensure the patency of blood flow at this position.

### Embodiment 3

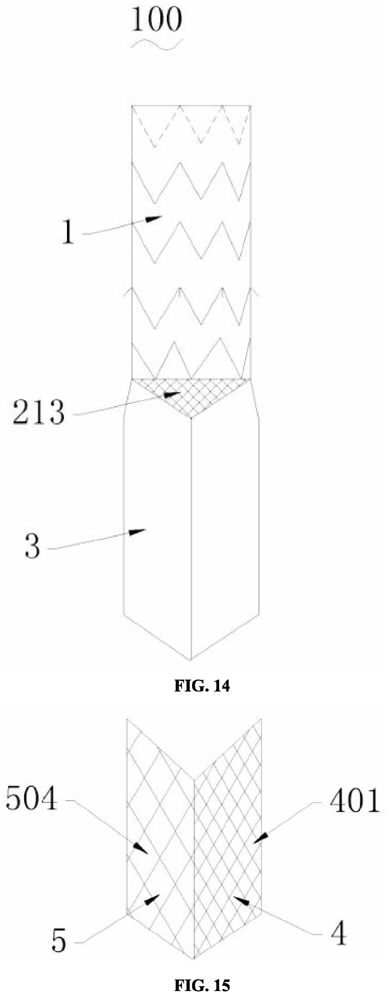

In this embodiment, referring to FIG. 15 to FIG. 17, structures of a main stent 10, a first embedded stent 4, and a second embedded stent 5 of the covered stent 100 are substantially the same as those in Embodiment 1, and a difference lies in that a first covering membrane 401 and a second covering membrane 504 are respectively arranged on surfaces of the first embedded stent 4 and the second embedded stent 5. To further ensure that the first embedded stent 4 and the second embedded stent 5 are connected to an aneurysm sac covered section 3 and then closely connected to a surface of a lumen of the aneurysm sac covered section 3 at proximal openings, and to avoid endoleaks between the first embedded stent 4 and the second embedded stent 5 and the aneurysm sac covered section 3, the first covering membrane 401 at least partially extends outward from the proximal opening of the first embedded stent 4 to form a connection portion 402, and the second covering membrane 504 at least partially extends outward from the proximal opening of the second embedded stent 5 to form a connection portion 402, the connection portions 402 form no-support covering membranes beyond the proximal openings of the first embedded stent 4 and the second embedded stent 5, and after the proximal openings of the first embedded stent 4 and the second embedded stent 5 are connected to a lumen wall of the aneurysm sac covered section 3 for the first time in a suturing or bonding manner along opening positions, the connection portion 402 of the first embedded stent 4 and the connection portion 402 of the second embedded stent 5 are connected to the aneurysm sac covered section 3 for the second time, to ensure that the proximal openings of the first embedded stent 4 and the second embedded stent 5 after connection are completely attached to the lumen of the aneurysm sac covered section 3 without a gap, wherein the first embedded stent 4 and the second embedded stent 5 are closely attached to each other side by side in the aneurysm sac covered section 3, and two attachment gaps 4021 are formed at close attachment tangent positions of the two embedded stents. Suturing at the gaps formed by the aneurysm sac covered section 3 and the first embedded stent 4 and the second embedded stent 5 often struggles to achieve a close attachment effect. Therefore, the connection portions 402 extending outward are at least arranged on one side of a mutual attachment position of the proximal openings of the first embedded stent 4 and the second embedded stent 5, to at least cover the two attachment gaps 4021 formed at the close attachment tangent positions of the two stents, and a better gap sealing effect is achieved through blockage by covering the covering membranes, instead of by directly connecting the aneurysm sac covered section 3.

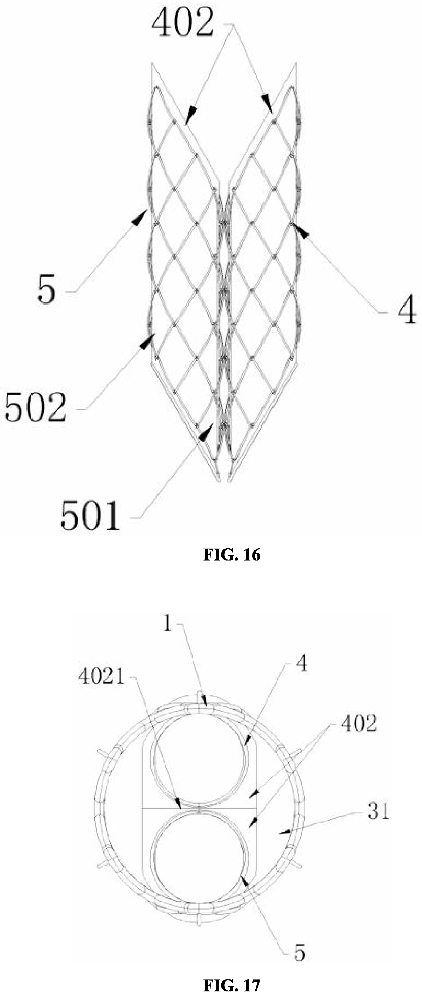

In some embodiments, continuing to refer to FIG. 17, the connection portions 402 extending outward are respectively arranged along the proximal openings of the first embedded stent 4 and the second embedded stent 5 in a circumferential direction. In this way, the two attachment gaps 4021 formed at the close attachment tangent positions of the first embedded stent 4 and the second embedded stent 5 can be sealed, and any position, connected to the aneurysm sac covered section 3, of the first embedded stent 4 and the second embedded stent 5 is further closed by secondary connection through the connection portions 402, to avoid endoleaks.

In this embodiment, the first covering membrane 401 and the second covering membrane 504 are made of materials different from a material of a surface covering membrane 31 of the main stent 10, and the surface covering membrane 31 is made of a polyethylene terephthalate (PET) material in this embodiment. Both the first covering membrane 401 and the second covering membrane 504 are expanded polytetrafluoroethylene (ePTFE) membranes. Herein, the PET membrane has the characteristic of high strength, while the ePTFE membrane has weak strength and smooth surface, is not easy to form thrombus, is good for long-term patency of small blood vessels, and has small pores. When the PET membrane and the ePTFE membrane are combined for use, not only the overall strength of the covering membrane on the main stent 10 is ensured, but also the first embedded stent 4 and the second embedded stent 5 have a better effect of isolating blood flow in the aneurysm sac covered section 3, to ensure the long-term patency of branches, that is, a good sealing effect.

In this embodiment, the arrangement of the second covering membrane 504 effectively isolates the first embedded stent 4 from the second embedded stent 5, such that a guidewire does not pass through the first embedded stent 4 or the second embedded stent 5 when introduced into the first embedded stent 4 or the second embedded stent 5, to ensure that the guidewire is accurately introduced into a corresponding lumen stent and avoid a problem that an implanted branch stent cannot reach an interior of a designated embedded stent.

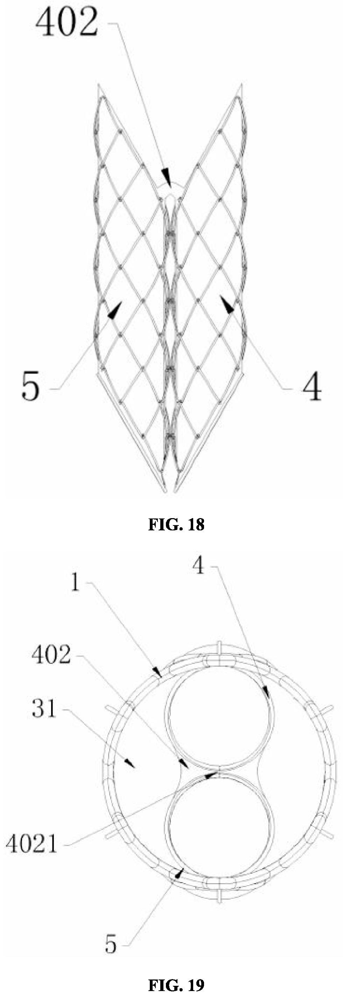

In another embodiment, referring to FIG. 18 and FIG. 19, in order to ensure the overall sealing integrity after the connection portions 402 are connected to the aneurysm sac covered section 3, the connection portion 402 of the first covering membrane 401 and the connection portion 402 of the second covering membrane 504 may be connected, and then the two connection portions 402 may be each connected to the aneurysm sac covered section 3. In this way, the proximal openings of the first embedded stent 4 and the second embedded stent 5 are integrated through the connection of the connection portions 402 first, and outer contours of the proximal openings of the two embedded stents are enclosed in circumferential directions of the proximal openings before the first embedded stent 4 and the second embedded stent 5 enter the aneurysm sac covered section 3 and are connected to the aneurysm sac covered section 3, such that after the first embedded stent 4 and the second embedded stent 5 are embedded in the aneurysm sac covered section 3, the proximal openings are connected for the first time, and the connection portions 402 are connected for the second time, thereby achieving a better edge sealing and endoleak preventing effect.

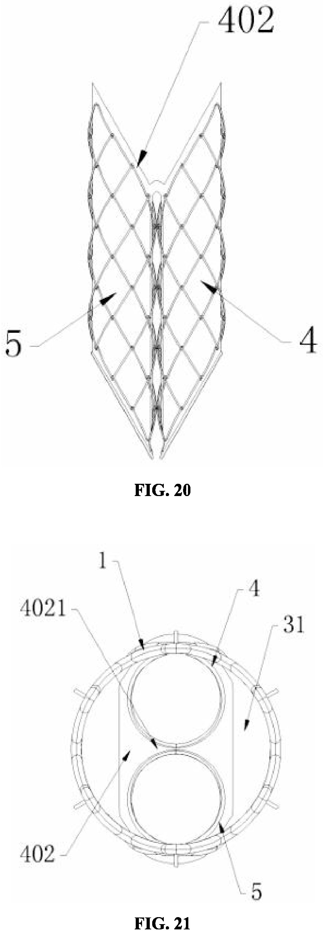

In some other embodiments, referring to FIG. 20 and FIG. 21, in order to further ensure the sealing integrity after the first embedded stent 4 and the second embedded stent 5 are connected to the aneurysm sac covered section 3 at the proximal openings to avoid endoleaks at attachment gaps 4021 between the first embedded stent 4 and the second embedded stent 5, a first covering membrane 401 and a second covering membrane 504 of the first embedded stent 4 and the second embedded stent 5 are integrally formed. Herein, integral forming means that the first covering membrane 401 and the second covering membrane 504 are continuously formed as a single covering membrane, without a bonding or suturing spliced structure. After forming, the first embedded stent 4 and the second embedded stent 5 which are respectively provided with the first covering membrane 401 and the second covering membrane 504 are integrally connected, and are connected together through the covering membranes on at least one side of a mutual attachment position of the proximal openings of the first embedded stent and the second embedded stent. Herein, since the first covering membrane 401 and the second covering membrane 504 are continuously formed as the single covering membrane, the first embedded stent and the second embedded stent are connected together through the covering membranes on one side of the mutual attachment position of the proximal openings, and the attachment gaps 4021 formed after the first embedded stent 4 and the second embedded stent 5 are attached to each other side by side are covered and blocked by the covering membranes, endoleaks do not occur at this position after connection to the aneurysm sac covered section, and blood is blocked by the covering membranes at this position. Since the first covering membrane 401 and the second covering membrane 504 are ePTFE membranes, an excellent blood isolation ability of the ePTFE membranes enhances an endoleak preventing effect at this position.

### Embodiment 4

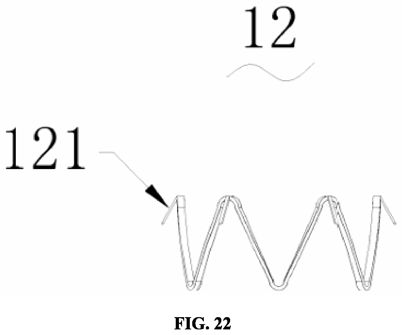

In this embodiment, referring to FIG. 1 and FIG. 22, structures of an aneurysm sac covered section 3, a first embedded stent 4, and a second embedded stent 5 of the covered stent 100 are substantially the same as those in Embodiment 1 to Embodiment 3, and a difference lies in that to enhance anchoring force of a proximal support section 1 in blood vessels or lumens of other stents, a support corrugated ring includes at least one anchoring corrugated ring. Herein, the support corrugated ring 11 of the proximal support section 1 may include only one anchoring corrugated ring 12, and a plurality of anchoring barbs 121 are circumferentially arranged on an outer side of the anchoring corrugated ring 12, to enhance an anchoring property of the proximal support section for vascular walls or inner side walls of stents.

In some embodiments, a plurality of support corrugated rings 11 on a surface covering membrane 31 of the proximal support section 1 are arranged in an axial direction, and the plurality of support corrugated rings 11 include at least one anchoring corrugated ring 12. The anchoring corrugated ring 12 is configured to provide better anchoring force between the proximal support section 1 and a blood vessel, wherein the anchoring corrugated ring 12 is arranged between a support corrugated ring 11 positioned at a proximal end portion of the proximal support section 1 and a support corrugated ring 11 positioned at a distal end portion, and a plurality of anchoring barbs 121 are circumferentially arranged on an outer side of the anchoring corrugated ring. Herein, the anchoring barbs 121 protrude an outer side wall of the proximal support section 1, and incline and extend toward a distal end. In this way, when the proximal support section 1 is released in the blood vessel, the support corrugated rings 11 provide support for expansion and anchoring force, and the anchoring barbs 121 on a middle support corrugated ring 11 pierce into a vascular wall to provide a more stable anchoring effect. Further, the anchoring corrugated ring 12 is arranged between the support corrugated ring 11 at the proximal end portion of the proximal support section 1 and the support corrugated ring 11 at the distal end portion, thereby enhancing the anchoring force near the middle of the proximal support section 1. In this way, whether from the middle to the proximal end or from the middle to the distal end, the anchoring corrugated ring 12 serving as a force-bearing point can ensure that both ends of the proximal support section 1 have sufficient connection force with the blood vessel. In some other embodiments, a plurality of anchoring corrugated rings 12 may be arranged, are located between a support corrugated ring 11 positioned at a proximal end portion and a support corrugated ring 11 positioned at a distal end portion, and may be located near the middle of the proximal support section 1, to further enhance the anchoring force of the proximal support section 1.

The design of the anchoring barbs 121 on the anchoring corrugated ring 12 ensures that since the anchoring barbs 121 enhance the anchoring force and the connection force, the covered stent 100 itself does not displace or shake, when an external iliac stent or an internal iliac stent is implanted into the covered stent 100 of the present invention after the proximal support section 1 of the covered stent 100 is implanted into a blood vessel or other stents.

### Embodiment 5

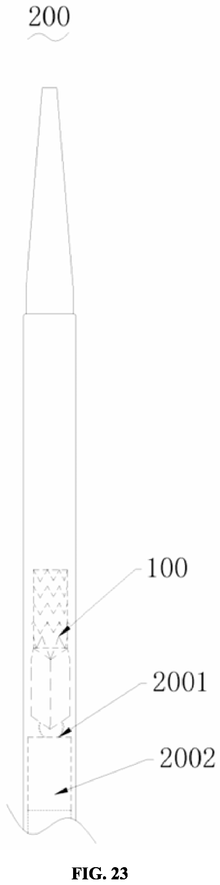

In this embodiment, referring to FIG. 23, structures of a main stent 10, a first embedded stent 4, and a second embedded stent 5 of the covered stent 100 are substantially the same as those in Embodiment 1 to Embodiment 4, and the embodiment also provides a stent delivery system 200, wherein the stent delivery system 200 includes the covered stent 100 as described in the foregoing embodiments and a delivery device, the delivery device is configured to deliver the covered stent 100 in the present invention to a designated vascular location and release the covered stent, the delivery device usually includes a delivery sheath and a delivery handle, the delivery handle is configured to control advancement and retraction of the delivery sheath to release the stent from the delivery sheath, the delivery device is also provided with a push rod 2002, a hook 2001 is connected to a proximal end of the push rod 2002, a hooking portion 503 of the covered stent 100 is configured to establish a connection with the hook 2001, and then the push rod 2002 controls to deliver the covered stent 100 to a relative position in the delivery sheath of the delivery device; the hook 2001 can be controlled by external force to switch between an unlocked state and a locked state, wherein when the hook 2001 is in the locked state, the hooking portion 503 and the hook 2001 are connected and cannot be detached; and when the hook 2001 is in the unlocked state, the hooking portion 503 can be separated from the hook 2001 for subsequent release of the covered stent 100.

The above-described specific embodiments are only part of embodiments of the present invention, and are not limitations to the present invention; and the specification does not enumerate all embodiments with the concept of the present invention. Furthermore, part of features from different embodiments described above may be interchanged or combined. Those skilled in the art may also make simple substitutions based on actual requirements. The concept of the present invention is subject to the scope of protection as specified.

## Claims

1. A covered stent, comprising a main stent provided with a tubular body, wherein the main stent comprises a proximal support section and an aneurysm sac covered section, and a distal end of the proximal support section is connected to a proximal end of the aneurysm sac covered section; the proximal support section comprises a support corrugated ring, the aneurysm sac covered section is internally provided with a first embedded stent and a second embedded stent arranged radially, a distal end of the aneurysm sac covered section is provided with an opening in communication with the outside, and distal openings of the first embedded stent and the second embedded stent are both in communication with the opening; and the first embedded stent has higher support strength than the second embedded stent.

2. The covered stent according to claim 1, wherein each of the first embedded stent and the second embedded stent comprises a mesh main body, and the first embedded stent has a larger wire diameter than the second embedded stent and/or a higher grid density than the second embedded stent on the mesh main body.

3. The covered stent according to claim 1, wherein distal ends of the first embedded stent and the second embedded stent are respectively provided with a first distal oblique opening and a second distal oblique opening, and the first distal oblique opening and the second distal oblique opening face away from each other.

4. The covered stent according to claim 3, wherein proximal ends of the first embedded stent and the second embedded stent are respectively provided with a first proximal flat opening and a second proximal flat opening; or proximal ends of the first embedded stent and the second embedded stent are respectively provided with a first proximal oblique opening and a second proximal oblique opening, and the first proximal oblique opening and the second proximal oblique opening face each other.

5. The covered stent according to claim 1, further comprising a transition section connected between the proximal support section and the aneurysm sac covered section, wherein a transition stent is arranged on the transition section.

6. The covered stent according to claim 1, wherein a first covering membrane is arranged on a surface of the first embedded stent, a second covering membrane is arranged on a surface of the second embedded stent, the first covering membrane and the second covering membrane at least partially extend outward from proximal openings of the first embedded stent and the second embedded stent respectively to form connection portions, and the connection portions are connected to the aneurysm sac covered section.

7. The covered stent according to claim 6, wherein the connection portion of the first covering membrane is connected to the connection portion of the second covering membrane.

8. The covered stent according to claim 7, wherein the first covering membrane and the second covering membrane are integrally formed.

9. The covered stent according to claim 3, wherein the first distal oblique opening and the second distal oblique opening each comprise a long-axis side wall and a short-axis side wall in a circumferential direction, an axial extension length of the long-axis side wall is greater than an axial extension length of the short-axis side wall, long-axis side walls of the first embedded stent and the second embedded stent are closely attached to each other, and a hooking portion is arranged at a distal end of at least one long-axis side wall.

10. The covered stent according to claim 1, wherein the support corrugated ring comprises at least one anchoring corrugated ring, and a plurality of anchoring barbs are circumferentially arranged on an outer side of the anchoring corrugated ring.

11. A stent delivery system, comprising the covered stent according to any one of claims 1 to 10.
